# EUROPEAN PATENT SPECIFICATION

(11) **EP 3 958 937 B1**
(45) Date of publication and mention of the grant of the patent: **28.08.2024**
(21) Application number: 20725918.5
(22) Date of filing: 16.04.2020
(51) Int. Cl.: A61M 15/00, A61M 15/08

(54) **ENDONASAL DRUG DELIVERY DEVICE**
VORRICHTUNG ZUR VERABREICHUNG EINES ENDONASALEM ARZNEIMITTELS
DISPOSITIF D'ADMINISTRATION DE MÉDICAMENT ENDONASAL

(30) Priority: 26.04.2019 IT 201900006368
(43) Date of publication of application: 02.03.2022
(73) Proprietor: HSD Technology SRL, 00195 Roma (RM) (IT)
(72) Inventor: NARCISO, Paolo, 00195 Roma (RM) (IT)
(74) Representative: Trentin, Michele
(86) International application number: PCT/IB2020/053594
(87) International publication number: WO 2020/217141

(56) References cited:
- WO-A1-2014/136135
- WO-A1-2016/180753
- WO-A1-2018/195086
- WO-A1-99/13930

## Description

### Field of application

The present invention is applicable to the health sector and has as particular object the intake of drugs by the respiratory route.

In more detail, the present invention relates to an endonasal drug delivery device.

### State of the art

In the field of health, techniques are known by which the intake of medicinal, therapeutic, and curative substances is performed by inhalation.

Inhalation is a technique for administering drugs by the respiratory route, typically carried out by means of devices for drugs intake in liquid, gaseous or solid powder form.

These administration techniques are particularly suitable for the treatment of respiratory problems.

For such purpose, devices known as aerosols are known, usually consisting of a nebulizer, i.e. an air compressor, which has the function of transforming drugs into a vehicle fluid that reaches the various organs of the respiratory system.

However, the presence of the compressing elements, which generate the air flow necessary for nebulization, entails bulky dimensions for these devices, making them unsuitable for portable use.

Furthermore, the characteristic nebulisation process of these devices is such as to require a long duration for the drugs intake. The timing required for therapies, together with the size of the devices, result in the need for users to remain still for the duration of the treatment.

In addition, these types of endonasal delivery devices involve significant waste of drugs by dispersion in the air, thereby reducing the effectiveness of the therapy.

According to the known art, to overcome these limits, there are portable inhalation devices in which the drugs, usually in liquid form, are contained in bottles which have an end shaped for introduction into the nasal cavities.

These devices allow immediate administration of the drugs, released into the user's nasal cavities during inspiration. The administering of the drugs takes place thanks to mechanically forced ventilation means, usually with the use of compressed gases.

However, although the dimensions are small compared to the devices which nebulise by means of a compressor, the known portable devices are still cumbersome and therefore inconvenient for transport.

Furthermore, the drug administration is not well controlled, leading to both a waste of substance and a difficult control of the correct dosage.

In fact, in endonasal delivery devices characterized by forced ventilation systems, the generated air flow enters the nasal cavity at a speed which is different than the normal respiratory flow speed; this results in a difficulty in combining the two air flows and consequently in a dispersion of the drugs.

Furthermore, the use of forced ventilation means involves difficulties in directing the generated flow, causing the drug particles to collide with areas not affected by the treatment and a further dispersion of substances.

Moreover, the use of forced ventilation means, due to the aforementioned problems of directing and mixing the air flow, implies endonasal delivery devices that are unsuitable for reaching the olfactory nerve and the terminal part of the trigeminal to perform a dispensation beyond the blood-brain barrier.

Furthermore, these devices are polluting the environment. In particular, they are polluting for the use of compressed gases and for the impossibility of reusing the drug reservoir.

Furthermore, these devices are suitable for the administration of liquid drugs, thus excluding the possibility of taking powdered drugs.

Patent documents WO 99/13930 A1, WO 2016/180753 A1, WO 2018/195086 A1 and WO 2014/136135 A1 are also known. They all describe devices in which there is an air flow induced by the breathing of the user who inhales drugs from a reservoir by perturbation.

These devices, despite the differences that distinguish them, are united by the fact that at least during the operational phase, the drug reservoir is the place where mixing takes place with the air flow that collect them due to disturbance. It follows a problematic control on the correct dosage of the administered drugs, since they are totally subjected to the air flow which, if excessively sustained, could collect them immediately and excessively.

Furthermore, such devices are forcibly of the disposable type since it is not possible to replace the drugs reservoir.

### Presentation of the invention

The object of the present invention is to provide an endonasal drug delivery device which allows to overcome at least partially the above highlighted drawbacks.

In particular, an object of the present invention is to provide an endonasal drug delivery device having compact size in order to be easily portable.

Another object of the present invention is to provide an endonasal delivery device which limits or cancels the drugs waste, ensuring the intake of a predetermined dosage.

A further object of the present invention is to provide an endonasal delivery device whose air flow is suitable for reaching the olfactory nerve and the terminal part of the trigeminal to carry out a dispensation beyond the blood-brain barrier.

Another object of the present invention is to provide an endonasal delivery device suitable for the administration of powdered drugs.

A further object of the present invention is to provide an endonasal delivery device which also allows the reuse of the drug reservoir.

Another object of the present invention is to provide an endonasal drug delivery device which involves reduced production costs compared to known equivalent devices.

A further object of the present invention is to provide an endonasal delivery device which does not require compressed gas or means for the forced inhalation of the drug.

Said objects, as well as others which will become clearer below, are achieved by an endonasal drug delivery device according to the following claims, which are to be considered as an integral part of this patent and which define the present invention.

The endonasal delivery device of the invention comprises a shaped body and a drug reservoir suitable for being inhaled by a patient.

According to the present invention, the shaped body is divisible into three portions.

A first portion is located at a first end of the shaped body and is shaped for insertion into the nasal cavity. It includes an air delivery duct to deliver air towards the nostril.

Then there is a second intermediate portion which contains an air supply duct to allow the inflow from the outside towards the inside of the endonasal delivery device.

The third portion is located at the opposite end of the first portion and comprises a mixing chamber. This chamber is in fluid communication with the supply duct, contained in the intermediate portion of the endonasal delivery device, so that air from the external environment can flow into it.

Furthermore, the mixing chamber is in fluid communication also with the aforementioned reservoir containing the drugs. This allows the drugs to flow into the chamber and mix by perturbation with the air coming from the external environment.

The air, thus enriched, proceeds in its flow (due to the normal breathing of the user) along the delivery duct, which is also in fluid communication with the mixing chamber, until it flows into the nasal cavity.

Advantageously, the form of the shaped body has limited dimensions such as to guarantee compactness to the endonasal drug delivery device.

Still advantageously, the drawing of the drugs by disturbance and their consequent delivery to the nasal cavities takes place by means of the air flow generated by the user's natural inspiration. In other words, the endonasal delivery device of the invention is advantageously passive, requiring no forced inhalation means such as compressors, fans or compressed gases. Therefore, this feature makes it possible not to resort on external elements which would entail a greater bulk.

Furthermore, still advantageously, the absence of forced ventilation means allows to take the drug by means of the air flow of natural respiration, improving the quality of inhalation and the directing of the air flow. These features allow to limit or cancel the drug waste ensuring a precise dosage.

Still advantageously, what has just been said allows the drug to reach the olfactory nerve and the terminal part of the trigeminal so as to perform a dispensation beyond the blood-brain barrier.

Still advantageously, the drawing of the medicinal substances from the mixing chamber by air disturbance makes the endonasal delivery device of the invention particularly suitable for the administration of powdered drugs.

Furthermore, the presence of a reservoir and a mixing chamber placed in fluid communication but independent one to the other, advantageously allows to operate a greater control on the correct dosage of the administered drug.

Still advantageously, the presence of an independent reservoir allows its replacement for a reuse of the endonasal delivery device that does not involve the exposure of the ducts to external contamination.

Furthermore, the possibility of replacing only the reservoir to allow reuse of the device advantageously reduces production costs with respect to known equivalent devices.

### Brief description of the drawings

Further features and advantages of the invention will be more evident in light of the detailed description of some preferred but not exclusive embodiments of an endonasal drug delivery device according to the invention, illustrated by way of non-limiting example with the aid of the accompanying drawings, wherein:

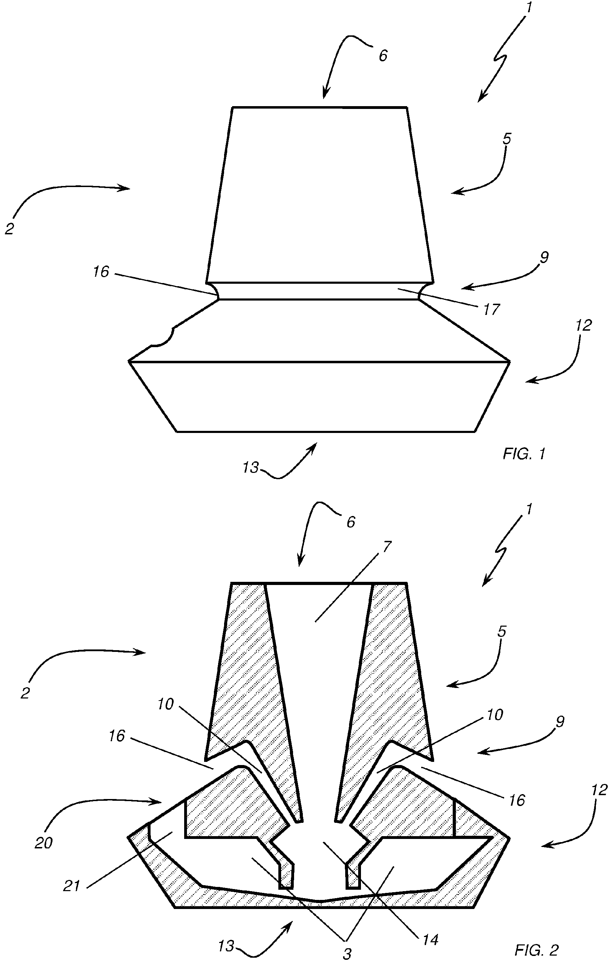

FIG. 1 represents a front view of the endonasal drug delivery device according to the invention;
FIG. 2 represents the endonasal delivery device of FIG. 1 in sectioned view.

### Detailed description of some preferred embodiments

With reference to the cited figures, an endonasal drug delivery device **1** according to the invention is described.

It comprises a shaped body **2** and a reservoir **3** of drugs suitable for being inhaled by a patient.

According to the invention, the shaped body **2** comprises a first portion 5 located at a first end **6** of the same shaped body **2.** This first portion **5** is shaped for insertion into the nasal cavity and includes an air delivery duct **7** to deliver air towards the nostril.

Obviously, such aspect must not be considered limiting for different embodiments of the invention, wherein, e.g., the first portion comprises multiple delivery ducts.

According to the invention, the shaped body **2** also comprises a second intermediate portion **9** which contains a supply duct **10** to allow the flow of air from the external environment towards the inside of the endonasal delivery device **1.**

In the figures, it is observed that the second portion **9** of the endonasal delivery device **1** comprises a plurality of supply ducts **10** arranged radially therein. Obviously, also these aspects must not be considered limiting for different embodiments of the invention wherein, for example, the number and the arrangement of the supply ducts are different and/or, according to other execution variants, such supply ducts are not totally included in the second intermediate portion.

According to the invention, the shaped body **2** comprises a third portion **12** located at the opposite end **13** to the first portion **5** and comprising a mixing chamber **14.**

The latter is in fluid communication with the supply duct **10,** contained in the intermediate portion **9** of the endonasal delivery device **1.** In this way, advantageously, the air from the external environment has the possibility of flowing to the aforementioned mixing chamber **14.**

Furthermore, the mixing chamber **14** is in fluid communication also with the reservoir **3** which, as can be seen from the figures, is separated from the mixing chamber **14** itself. From the reservoir **3** the drugs can then flow into the chamber **14** and be mixed by perturbation with the air coming from the external environment.

Finally, the delivery duct **7** is also in fluid communication with the chamber **14.** The fluid communication between the supply duct **10,** the mixing chamber **14** and the delivery duct **7** creates a channel by which air can flow between the external environment and the nasal cavities allowing normal patient breathing. Advantageously, however, in the case of the present invention, the air that flows to the nasal cavities by means of the delivery duct **7** is that enriched with the drugs in the mixing chamber **14.**

Still advantageously, the drawing by disturbance of the drugs and their consequent inflow into the nasal cavities takes place by means of an air flow generated by the user's natural inspiration. It is therefore not necessary to use special means of forced ventilation such as compressed gases, fans or compressors. This makes the endonasal delivery device **1** of the invention passive, reducing its size compared to the active devices of the state of the art.

Moreover, still advantageously, the intake of drugs by natural respiration improves the quality of inhalation allowing a more effective directing of the air flow since, among other things, the problems with combining two air flows with different characteristics are avoided (the one forced and that of natural breathing). In particular, the drawing of drugs by disturbance with a natural respiratory flow improves the effectiveness of mixing between the flow itself and the drugs, limiting, if not eliminating, the drugs waste and thus allowing the intake of a precise dosage of drugs.

Still advantageously, the intake of drugs by means of an air flow generated by natural respiration, without the need to resort to forced ventilation means, allows to reach the olfactory nerve and the terminal part of the trigeminal in order to carry out a dispensation beyond the blood-brain barrier.

The drawing of the drug by disturbance allows, still advantageously, to make the endonasal delivery device **1** of the invention particularly suitable for the administration of drugs in powder form.

Also the production costs of the endonasal delivery device **1** of the invention are evidently reduced compared to the active devices just mentioned.

Still advantageously, the compactness of the endonasal delivery device **1** of the invention makes it easily and comfortably transportable and usable anywhere.

Furthermore, the presence of a reservoir **3** and a mixing chamber **14** placed in fluid communication but independent one to the other advantageously allows greater control over the correct dosage of the administered drugs.

According to the embodiment of the invention which is described, the intermediate portion **9** also includes openings **16** along the lateral surface **17.** In particular, the openings **16** are each arranged at a respective supply duct **10,** defining a connection between the external environment and the interior of the endonasal delivery device **1.**

Advantageously, the openings **16** connect the fluid communication channel created between the supply ducts **10,** the mixing chamber **14** and the delivery channel 7 with the outside, allowing the air flow to draw the drugs by disturbance and reach the nasal cavities.

Obviously, these aspects should not be considered limitative for different embodiments of the invention, wherein, e.g., the opening to the outside is unique and consists of a circumferential groove to the shaped body and communicating with the plurality of supply ducts. Furthermore, according to other embodiments, the openings are made in the third portion as well as a part of the supply ducts.

As regards the reservoir **3,** according to the embodiment which is described it is contained within the third portion **12.**

Advantageously, the insertion of the reservoir **3** inside the shaped body **2** allows to further reduce the dimensions of the endonasal delivery device **1,** thereby increasing its compactness and the ease of transport.

Obviously, also this aspect must not be considered limiting for different embodiments of the invention, wherein, for example, the reservoir is placed outside the shaped body or inside one of the other portions of the body itself.

In particular, according to a different embodiment of the invention, the reservoir is inserted in disposable capsules separated from the shaped body. These capsules are shaped to be removably attached to the endonasal delivery device typically outside the third portion of the shaped body. They are also placed in fluid communication with the mixing chamber so as to allow the drawing of the drug by disturbance.

According to this variant, the capsules contain the correct dosage of drug to be inhaled allowing the perfect dosage. They also advantageously allow the same shaped body to be used for the inhalation of different drugs.

Returning to the embodiment of the invention represented in the figures, the reservoir **3** is equipped with an inlet **20.** In more detail, such inlet **20** preferably, but not necessarily, consists of an intake duct **21** through which it is possible to introduce the drug to be taken into the reservoir **3.**

Advantageously, following the administering of the drugs and the consequent emptying of the reservoir **3,** through the opening **20** the patient can deposit a new drugs dose and reuse the endonasal delivery device **1** with the same effectiveness as the first treatment.

Still advantageously, in this way it is possible to control the exact quantity of drug to be taken avoiding incorrect dosages.

Operatively, the patient prepares the endonasal delivery device **1** by inserting the predetermined dose of drug into the reservoir **3** through the inlet **20.** Then he inserts the first portion **5** of the endonasal delivery device **1** into the nasal cavity and proceeds to the drug intake simply by breathing.

The air flow, inspired by the patient, enters the endonasal delivery device **1** through the openings **16** placed on the lateral surface **17** of the shaped body **2.** Subsequently, the air flows through the supply ducts **10** and flows into the mixing chamber **14.** Inside the latter, the air mixes by disturbance with the drugs that have flowed from the reservoir **3.** Then, the air enriched with the drugs travels through the delivery duct **7** until pouring into the nasal cavity of the patient. The administration of the pre-established dose of drugs requires several breathing cycles. At the end of the intake, the patient removes the endonasal delivery device **1** from the nasal cavity.

Subsequently, at the time of a new drug intake, the patient reuses the same endonasal delivery device **1** and, similarly to what was previously done, he fills the reservoir **3** again with the predetermined drug dosage.

In light of the foregoing, it is understood that the endonasal drug delivery device of the invention achieves all the prefixed purposes.

In particular, it is compact enough to make it easily and comfortably transportable and usable anywhere.

On closer inspection, however, the flow of air is generated by the user's natural inspiration, without the need to resort to special means of forced ventilation such as compressed gases, fans or compressors, improving the directing of the air flow and the quality of inhalation.

Furthermore, the drugs intake by means of an air flow generated by natural respiration allows to reach the olfactory nerve and the terminal part of the trigeminal to carry out a dispensation beyond the blood-brain barrier.

It is also noted that the drawing of drugs by disturbance allows to improve the mixing between the air flow and the drugs, allowing to limit, if not cancel, the drugs waste during the intake, thus allowing to take precise dosages of drugs.

In addition, the removal of the drug due to disturbance allows the administration of powdered drugs.

In addition, the reservoir is equipped with an inlet for the introduction of drugs, thus allowing the reuse of the endonasal delivery device and the correct dosage of drug to be taken.

Finally, as far as has been said so far, it is clear that the production costs are reduced compared to the active devices belonging to the state of the art.

The invention might be subject to many changes and variants, which are all included in the appended claims. Moreover, all the details may furthermore be replaced by other technically equivalent elements, and the materials may be different depending on the needs, without departing from the protection scope of the invention defined by the appended claims.

## Claims

1. Endonasal drug delivery device (1) suitable for inhalation by a patient comprising a shaped body **(2)** having:
- at least one first portion **(5)** located at a first end **(6)** of said shaped body **(2),** shaped to be inserted in a nasal cavity and having at least one air delivery duct **(7)** towards the nasal cavity;
- at least one second intermediate portion **(9)** having at least one supply duct **(10)** to allow the air inflow from the outside towards the inside of said endonasal delivery device **(1);**
- at least one third portion **(12)** located at the opposite end **(13)** of said first portion **(5)** and comprising at least one mixing chamber **(14),**
wherein said mixing chamber **(14)** is in fluid communication with:
- said at least one supply duct **(10)** to receive air from the outside;
- at least one drug reservoir **(3)** separated from said mixing chamber **(14)** and in fluid connection with it to allow the drugs to flow into said mixing chamber **(14)** to be drawn from the air by disturbance;
- said at least one delivery duct **(7)** for allowing the flow of drugs mixed with air into the nasal cavity,
said fluid communication identifying a fluidic path that provides the air to inflow from the outside towards the inside of said endonasal delivery device **(1)** by said supply duct (10), then walking towards said one mixing chamber **(14),** then collect the drugs by disturbance, so the drugs flow to said mixing chamber **(14)** by the pressure differential between said mixing chamber **(14)** and said reservoir **(3)** and finally the mix air-drugs flows towards said delivery duct (7) towards the nasal cavity.

2. Endonasal drug delivery device according to claim 1, wherein said reservoir **(3)** is incorporated in said third portion (**12**).

3. Endonasal drug delivery device according to claim 1 or 2, wherein said reservoir **(3)** has an inlet **(20)** for the introduction of the drugs to be inhaled.

4. Endonasal drug delivery device according to claim 3, wherein said inlet **(20)** is constituted by an intake duct **(21).**

5. Endonasal drug delivery device according to claim 1, wherein said reservoir is arranged externally to said third portion and fluidly coupled to it.

6. Endonasal drug delivery device according to any of the preceding claims, wherein said intermediate portion **(9)** has at least one opening **(16)** arranged on the lateral surface **(17)** of said shaped body (2) and communicating with said supply duct **(10).**

7. Endonasal drug delivery device according to any one of claims 1 to 5, wherein said intermediate portion comprises a plurality of said supply ducts radially arranged in said intermediate portion.

8. Endonasal drug delivery device according to claim 7, wherein said intermediate portion has a plurality of openings each corresponding to a said supply duct.

## Patentansprüche

1. Endonasale Arzneimittelzuführvorrichtung (1), die für die Inhalation durch einen Patienten geeignet ist, umfassend einen geformten Körper (2), der folgendes aufweist:
- mindestens einen ersten Abschnitt (5), der sich an einem ersten Ende (6) des geformten Körpers (2) befindet, zum Einführen in die Nasenhöhle geformt ist, und mindestens einen Luftzuführkanal (7) in Richtung der Nasenhöhle aufweist;
- mindestens einen zweiten Mittelabschnitt (9), der mindestens einen Versorgungskanal (10) aufweist, um die Lufteinströmung von der Außenseite zu der Innenseite der endonasalen Zuführvorrichtung (1) zuzulassen;
- mindestens einen dritten Abschnitt (12), der sich an dem Gegenende (13) des ersten Abschnitts (5) befindet und mindestens eine Mischkammer (14) umfasst,
wobei die Mischkammer (14) sich in Fließkommunikation befindet mit:
- dem mindestens einen Versorgungskanal (10), um Luft von der Außenseite zu empfangen;
- mindestens einem Arzneimittelreservoir (3), das getrennt von der Mischkammer (14) und in Fließverbindung damit vorliegt, um zu ermöglichen, dass Arzneimittel, die in die Mischkammer (14) strömen sollen, von der Luft mittels Störung gezogen werden;
- dem mindestens einen Zuführkanal (7), um die Strömung von Arzneimitteln, gemischt mit Luft, in die Nasenhöhle zuzulassen,
wobei die Fließkommunikation einen Fließpfad identifiziert, der die Luft zum Einströmen von der Außenseite zu der Innenseite der endonasalen Zuführvorrichtung (1) durch den Versorgungskanal (10) bereitstellt, dann zu der einen Mischkammer (14) geht, dann die Arzneimittel durch Störung einsammelt, damit die Arzneimittel durch die Druckdifferenz zwischen der Mischkammer (14) und dem Reservoir (3) zu der Mischkammer (14) strömen, und schließlich die Mischung aus Luft und Arzneimitteln zu dem Zuführkanal (7) zu der Nasenhöhle strömt.

2. Endonasale Arzneimittelzuführvorrichtung nach Anspruch 1, wobei das Reservoir (3) in den dritten Abschnitt (12) eingebaut ist.

3. Endonasale Arzneimittelzuführvorrichtung nach Anspruch 1 oder 2, wobei das Reservoir (3) einen Einlass (20) zur Einbringung der zu inhalierenden Arzneimittel aufweist.

4. Endonasale Arzneimittelzuführvorrichtung nach Anspruch 3, wobei der Einlass (20) durch einen Einnahmekanal (21) gebildet wird.

5. Endonasale Arzneimittelzuführvorrichtung nach Anspruch 1, wobei das Reservoir außerhalb des dritten Abschnitts angeordnet und fließtechnisch daran gekoppelt ist.

6. Endonasale Arzneimittelzuführvorrichtung nach einem der vorhergehenden Ansprüche, wobei der Zwischenabschnitt (9) mindestens eine Öffnung (16) aufweist, die auf der lateralen Oberfläche (17) des geformten Körpers (2) angeordnet ist und mit dem Versorgungskanal (10) kommuniziert.

7. Endonasale Arzneimittelzuführvorrichtung nach einem der Ansprüche 1 bis 5, wobei der Zwischenabschnitt eine Vielzahl der Versorgungskanäle umfasst, die in dem Zwischenabschnitt radial angeordnet sind.

8. Endonasale Arzneimittelzuführvorrichtung nach Anspruch 7, wobei der Zwischenabschnitt eine Vielzahl von Öffnungen hat, von denen jede einem der Versorgungskanäle entspricht.

## Revendications

1. Dispositif d'administration de médicament par voie endonasale (1) adapté à l'inhalation par un patient, comprenant un corps façonné **(2)** présentant :
- au moins une première partie **(5)** située à une première extrémité **(6)** dudit corps façonné **(2),** façonnée pour être insérée dans une cavité nasale et présentant au moins un conduit d'acheminement d'air **(7)** vers la cavité nasale ;
- au moins une deuxième partie intermédiaire **(9)** présentant au moins un conduit d'alimentation **(10)** pour permettre l'arrivée d'air de l'extérieur vers l'intérieur dudit dispositif d'administration endonasale **(1)** ;
- au moins une troisième partie **(12)** située à l'extrémité opposée **(13)** de ladite première partie **(5)** et comprenant au moins une chambre de mélange **(14),**
dans lequel ladite chambre de mélange **(14)** est en communication fluidique avec :
- ledit au moins un conduit d'alimentation **(10)** pour recevoir de l'air de l'extérieur ;
- au moins un réservoir de médicament **(3)** séparé de ladite chambre de mélange **(14)** et en liaison fluidique avec elle pour permettre aux médicaments de s'écouler dans ladite chambre de mélange **(14)** afin d'être aspirés depuis l'air par une perturbation ;
- ledit au moins un conduit d'acheminement **(7)** permettant l'écoulement de médicaments mélangés à de l'air dans la cavité nasale,
ladite communication fluidique identifiant un chemin fluidique qui permet à l'air d'affluer de l'extérieur vers l'intérieur dudit dispositif d'administration par voie endonasale **(1)** par ledit conduit d'alimentation (10), puis de se diriger vers ladite chambre de mélange **(14),** puis recueillant les médicaments par perturbation, de sorte que les médicaments s'écoulent vers ladite chambre de mélange **(14)** par la différence de pression entre ladite chambre de mélange **(14)** et ledit réservoir **(3)** et enfin le mélange air-médicaments s'écoule vers ledit conduit d'acheminement **(7)** vers la cavité nasale.

2. Dispositif d'administration de médicament par voie endonasale selon la revendication 1, dans lequel ledit réservoir **(3)** est incorporé dans ladite troisième partie **(12).**

3. Dispositif d'administration de médicament par voie endonasale selon la revendication 1 ou 1, dans lequel ledit réservoir **(3)** présente une entrée **(20)** pour l'introduction des médicaments à inhaler.

4. Dispositif d'administration de médicament par voie endonasale selon la revendication 3,
dans lequel ladite entrée **(20)** est constituée par un conduit d'admission **(21).**

5. Dispositif d'administration de médicament par voie endonasale selon la revendication 1, dans lequel ledit réservoir est agencé à l'extérieur de ladite troisième partie et couplé fluidiquement à celle-ci.

6. Dispositif d'administration de médicament par voie endonasale selon l'une quelconque des revendications précédentes, dans lequel ladite partie intermédiaire **(9)** présente au moins une ouverture **(16)** agencée sur la surface latérale **(17)** dudit corps façonné **(2)** et communiquant avec ledit conduit d'alimentation **(10).**

7. Dispositif d'administration de médicament par voie endonasale selon l'une quelconque des revendications 1 à 5, dans lequel ladite partie intermédiaire comprend une pluralité desdits conduits d'alimentation agencés radialement dans ladite partie intermédiaire.

8. Dispositif d'administration de médicament par voie endonasale selon la revendication 7, dans lequel ladite partie intermédiaire présente une pluralité d'ouvertures correspondant chacune à un dit conduit d'alimentation.
